(19) Europäisches Patentamt
European Patent Office
Office européen des brevets

(11) Veröffentlichungsnummer: **0 144 749**
**A2**

# (12) EUROPÄISCHE PATENTANMELDUNG

(21) Anmeldenummer: **84113260.8**

(22) Anmeldetag: **03.11.84**

(51) Int. Cl.[4]: **C 07 D 233/70**
C 07 D 235/02, C 07 D 405/12
C 07 D 403/12, A 01 N 43/50

(30) Priorität: **10.11.83 DE 3340594**

(43) Veröffentlichungstag der Anmeldung:
**19.06.85 Patentblatt 85/25**

(84) Benannte Vertragsstaaten:
**AT BE CH DE FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Schmierer, Roland, Dr.**
**An der Weinleite 5a**
**D-8901 Todtenweis(DE)**

(72) Erfinder: **Handte, Reinhard, Dr.**
**Theilweg 23**
**D-8901 Gablingen(DE)**

(72) Erfinder: **Mildenberger, Hilmar, Dr.**
**Fasanenstrasse 24**
**D-6233 Kelkheim (Taunus)(DE)**

(72) Erfinder: **Bauer, Klaus, Dr.**
**Kolpingstrasse 7**
**D-6054 Rodgau(DE)**

(72) Erfinder: **Bieringer, Hermann, Dr.**
**Eichenweg 26**
**D-6239 Eppstein/Taunus(DE)**

(72) Erfinder: **Bürstell, Helmut, Dr.**
**Am Hohlacker 65**
**D-6000 Frankfurt am Main 50(DE)**

(54) **2-acrlsubstituierte Imidazolinone, Verfahren zu ihrer Herstellung und ihre Verwendung im Pflanzenschutz.**

(57) 2-Arylimidazolinone der Formel

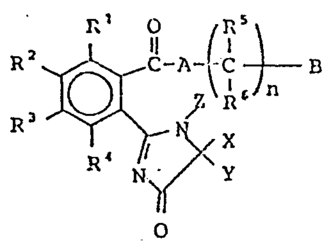

1

worin A O, S, NH oder N-Alkyl; B u.a. CN, eine Carbonester-, Carbothioester- oder Carbonamidgruppe; X Alkyl, Y Alkyl, Cycloalkyl, Alkenyl, Alkinyl, Phenyl oder Benzyl oder X und Y zusammen mit C eine Spiro-cycloalkylgruppe; Z H, (subst.) Alkyl, Alkenyl, Propargyl, eine Acyl-, Carbonester- oder Sulfoestergruppe; n 1 - 3; $R^1$ - $R^4$ Alkyl, Alkoxy, Alkoxycarbonyl, Halogen, $No_2$, CN, (subst.) Phenoxy, (subst.) Phenyl oder je zwei benachbarte den Rest $-CH=CH-CH=CH-$; und $R^5$ und $R^6$ H, Alkyl oder Phenyl darstellen, sind wirksame Herbizide und Wachstumsregulatoren.

EP 0 144 749 A2

Croydon Printing Company Ltd.

Patentansprüche für Österreich:

1. Verfahren zur Herstellung von Imidazolinonen der Formel 1

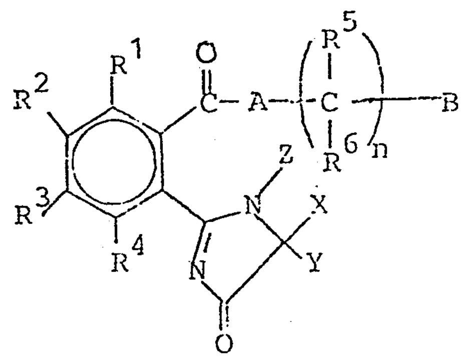

1

in welcher

A O, S, NH oder $N(C_1-C_6)$Alkyl;

B -CN, $-COOR^7$, $-CONR^8R^9$ oder $-COSR^{10}$;

X $(C_1-C_4)$Alkyl;

Y $(C_1-C_6)$Alkyl, Cyclo$(C_3-C_6)$alkyl, $(C_2-C_4)$Alkenyl, $(C_2-C_4)$Alkinyl, Phenyl oder Benzyl;

X und Y zusammen mit dem Kohlenstoffatom, an dem sie gebunden sind, eine gegebenenfalls durch $-CH_3$ substituierte Spiro$(C_3-C_6)$cycloalkylgruppe;

Z Wasserstoff, $(C_1-C_4)$Alkyl, welches durch $(C_1-C_4)$Alkoxycarbonyl substituiert sein kann, $(C_3-C_4)$Alkenyl, Propargyl, $-CO-R^{11}$ oder $-SO_2-R^{12}$;

n die Zahlen 1, 2 oder 3;

$R^1$, $R^2$, $R^3$ und $R^4$ unabhängig voneinander Wasserstoff, $(C_1-C_4)$Alkyl, $(C_1-C_6)$Alkoxy, $(C_1-C_6)$Alkoxycarbonyl, Halogen, Nitro, Cyano, Phenoxy oder Phenyl, das gegebenenfalls mit $(C_1-C_4)$Alkyl, $(C_1-C_4)$Alkoxy oder Halogen substituiert ist, und jeweils zwei benachbarte Reste $R^1$, $R^2$, $R^3$ oder $R^4$ gemeinsam die Gruppierung -CH=CH-CH=CH- bilden können;

$R^5$ und $R^6$ unabhängig voneinander Wasserstoff, $(C_1-C_4)$-Alkyl oder Phenyl;

$R^7$ Wasserstoff, $(C_1-C_{12})$Alkyl, welches gegebenenfalls bis zu zweimal, vorzugsweise jedoch einfach mit $(C_1-C_4)$Alkoxy, $(C_1-C_4)$Alkoxyethoxy, Cyclo$(C_3-C_6)$alkyl, Benzyloxy, Phenyl, Halogen, Cyano, Hydroxyl, Oxiranyl, Tetrahydrofuryl, $(C_1-C_4)$Alkylamino, Di$(C_1-C_4)$alkylamino, $(C_1-C_4)$Alkylthio, $(C_1-C_4)$Alkylsulfinyl, $(C_1-C_4)$Alkylsulfonyl, Triazolyl oder Imidazolyl substituiert ist; $(C_3-C_6)$Alkenyl, Halogen$(C_3-C_6)$-alkenyl, Cyclo$(C_3-C_6)$alkyl, Cyclo$(C_5-C_6)$alkenyl, $(C_3-C_6)$Alkinyl, Benzyl, welches gegebenenfalls im Benzolkern durch Chlor oder Methyl substituiert sein kann, Phenyl, welches gegebenenfalls mit bis zu zwei $(C_1-C_4)$Alkyl-, Nitro-, Halogen- oder Methoxygruppen substituiert sein kann, oder die Gruppe

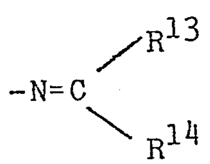

$R^8$ Wasserstoff oder $(C_1-C_4)$Alkyl;

$R^9$ Wasserstoff, $(C_1-C_6)$Alkyl, welches gegebenenfalls mit einer Hydroxygruppe oder einer $(C_1-C_3)$Alkoxygruppe substituiert ist, $(C_1-C_4)$Alkylsulfonyl oder Halogen$(C_1-C_4)$alkylsulfonyl;

$R^8$ und $R^9$ gemeinsam mit dem Stickstoffatom einen 5- oder 6-gliedrigen Ring, in dem ein Kohlenstoffatom durch Sauerstoff oder Stickstoff ersetzt sein kann und der gegebenenfalls mit bis zu zwei Methylgruppen substituiert ist;

$R^{10}$ ($C_1$-$C_6$)Alkyl, Phenyl oder ($C_1$-$C_4$)Alkoxycarbonyl-($C_1$-$C_2$)alkyl;

$R^{11}$ ($C_1$-$C_{12}$)Alkyl, das gegebenenfalls mit bis zu zwei ($C_1$-$C_4$)Alkoxygruppen oder mit bis zu drei Halogen substituiert ist; Phenyl, das mit bis zu zwei Halogen, einer Methyl-, einer Nitro- oder einer Methoxygruppe substituiert sein kann; ($C_1$-$C_4$)Alkoxy, Cyclo($C_3$-$C_7$)alkyl, ($C_1$-$C_4$)Alkoxycarbonyl, Benzyloxy, Phenoxy oder $-NR^{15}R^{16}$;

$R^{12}$ ($C_1$-$C_4$)Alkyl, $CF_3$, $CCl_3$, Phenyl, Chlorphenyl oder Methylphenyl;

$R^{13}$ und $R^{14}$ unabhängig voneinander Wasserstoff, ($C_1$-$C_4$)-Alkyl, ($C_1$-$C_4$)Alkoxy($C_1$-$C_4$)alkyl, Cyclo($C_3$-$C_6$)-alkyl oder Phenyl, oder gemeinsam mit dem Kohlenstoffatom, an dem sie gebunden sind, eine Cyclo($C_5$-$C_6$)alkylgruppe;

$R^{15}$ Wasserstoff oder ($C_1$-$C_4$)Alkyl, und

$R^{16}$ ($C_1$-$C_4$)Alkyl, Phenyl, Chlorphenyl, Methylphenyl, Amino, Mono- oder Di($C_1$-$C_4$)alkylamino

bedeuten, sowie deren optische Isomeren (falls $X \neq Y$ und/oder $R^5 \neq R^6$) sowie ihre Salze mit Säuren und Basen.

$R^7$ und $R^{11}$ bedeuten insbesondere ($C_1$-$C_{12}$)Alkyl, bevorzugt ($C_1$-$C_4$)Alkyl.

Man erhält die erfindungsgemäßen Verbindungen der allgemeinen Formel 1, indem man

a) Amide der Formel 2

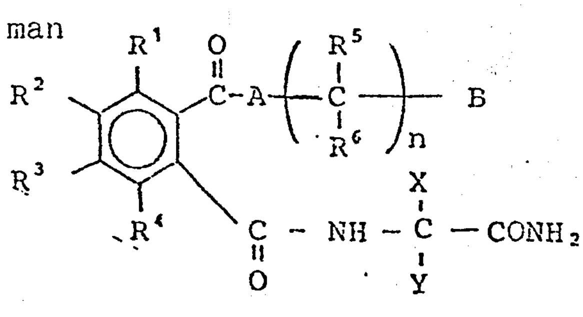

2

durch Wasserabspaltung cyclisiert oder

b) Verbindungen der Formel 3,

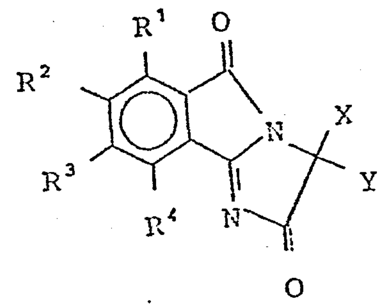

3

mit mindestens äquimolaren Mengen der Verbindungen der Formel 4

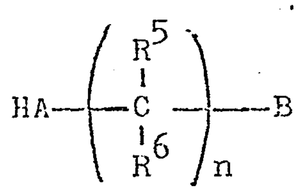

4

umsetzt

und gewünschtenfalls die erhaltenen Verbindungen der Formel 1 mit Z= Wasserstoff durch Alkylierung, Acylierung, Sulfonierung, Oxidation, Verseifung, Veresterung oder Salzbildung in andere Verbindungen der Formel 1 überführt.

Zu a): Die Cyclisierung der Amide 2 kann z.B. mit Phosphorpentachlorid, vorteilhaft in Gegenwart eines unter den Reaktionsbedingungen inerten Lösemittels erfolgen. Als Beispiele seien genannt: Toluol, Xylol, Chloroform oder Phosphoroxychlorid. Die Reaktionstemperatur ist unkritisch und kann zwischen -10°C und +150°C variiert werden. Besonders vorteilhaft sind Reaktionstemperaturen zwischen 0 und 100°C. Man erhält dabei primär die Hydrochloride der Imidazolinone 1. Nach üblichen Methoden, z.B. durch Umsetzung mit Natriumcarbonat oder Natriumhydrogencarbonat, lassen sich daraus die freien Basen herstellen.

Die Cyclisierung kann ebenfalls in Gegenwart starker organischer oder anorganischer Säuren, wie Schwefelsäure, oder p-Toluolsulfonsäure, unter gleichzeitiger Abtrennung des gebildeten Wassers bei Temperaturen von 0°C bis 150°C erfolgen. Besonders vorteilhaft läßt sich die Reaktion so führen, daß das gebildete Wasser durch Azetropdestillation mit einem Lösemittel, wie Toluol, Xylol oder Chloroform, abgetrennt wird. Nach Neutralisation können die Produkte nach üblichen Methoden isoliert werden.

Zu b): Die Umsetzung läßt sich vorteilhaft in Gegenwart eines inerten Lösungsmittels, wie Toluol oder Chloroform durchführen, jedoch kann auch ein Überschuß der Reaktionskomponente 4 selbst als Lösemittel dienen. Der Zusatz einer anorganischen oder organischen Base wirkt sich in vielen Fällen vorteilhaft aus. Die Reaktionstemperatur für diese Umsetzung liegt im Bereich zwischen 0°C und +150°C, vorteilhaft zwischen +20°C und 110°C.

Die Reaktionsprodukte der Formel 1 mit Z= Wasserstoff können in einfacher Weise nach an sich bekannten Verfahren durch Umsetzung mit Alkylierungsmitteln (Methyljodid, Dimethylsulfat) oder Acylierungsmitteln (Säurechloriden) in Gegenwart von Basen oder mit Isocyanaten zu den Verbindungen der Formel 1 mit Z= Alkyl, Alkenyl, Propargyl, $-COR^{11}$ oder $-SO_2-R^{12}$, umgesetzt werden.

Verbindungen der Formel 1 in den $R^7$ für Niederalkyl steht, können auch nach geläufigen Verfahren, wie z.B. Verseifung, Umesterung, Amidierung, in andere Verbindungen der Formel 1, umgewandelt werden.

Die erfindungsgemäßen Verbindungen sind, wenn Z für Wasserstoff steht, tautomer, so daß sie in einer der beiden Formen 1a / 1b oder als Gemisch von 1a und 1b vorliegen können. Diese Isomeren treten auch bei den Derivaten mit $Z \neq H$ auf.

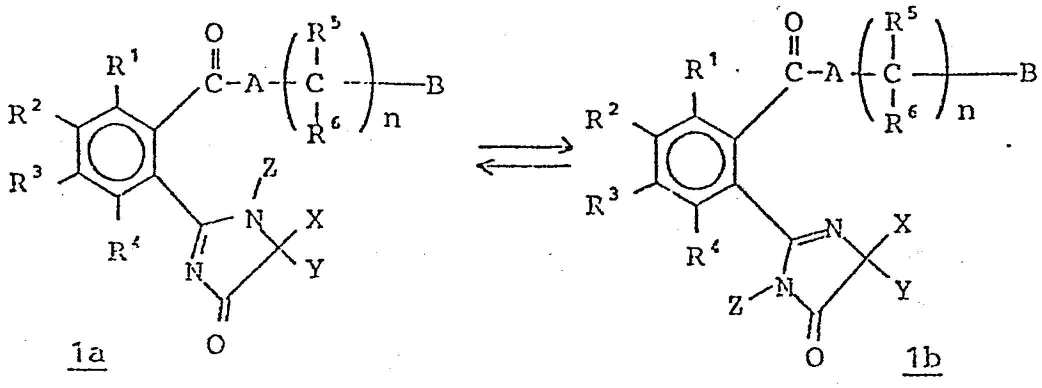

1a 1b

Die Definitionen der Formel 1 umfassen stets beide isomeren Strukturen der Formeln 1a und 1b.

Die Säureadditionssalze der Verbindungen der Formel 1 bzw. die Salze mit organischen oder anorganischen Basen sind auf allgemein bekanntem Weg gut zugänglich. Zur Salzbildung sind einerseits Säuren wie $H_2SO_4$, HCl, $HNO_3$ oder Essigsäure, zum anderen Basen wie NaOH, KOH, Pyridin, Triethylamin, Tris-hydroxyethylamin, $NH_3$ udgl. befähigt.

Die Amide der Formel 2 lassen sich leicht aus den Aminoamiden 5 und den entsprechend substituierten Carbonsäurederivaten 6 erhalten. Als Derivate geeignet sind z. B. Säurechloride oder Kohlensäurealkylester ($R^{17}$ = Cl, -O-COOAlkyl).

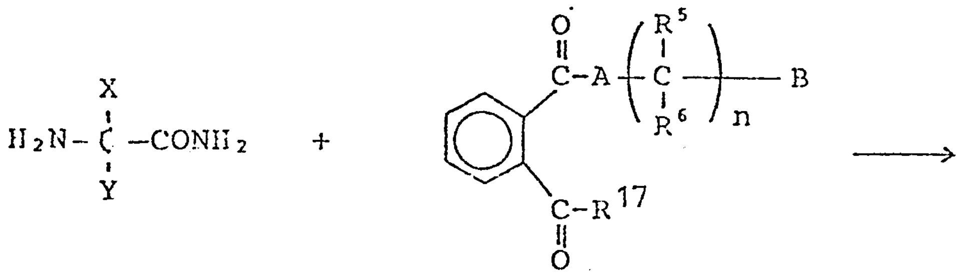

2

5 6

Verbindungen der Formel 3 sind nach dem in der DE-A 27 00 270 angegebenen Verfahren zugänglich.

Werden für die Synthese der erfindungsgemäßen Verbindungen der Formel 1 als Ausgangsstoffe unsymmetrisch substituierte Phthalsäureanhydride eingesetzt, so können sowohl bei den Amiden der Formel 2 als auch bei den Verbindungen der Formel 3, die in den Verfahrensvarianten a) und b) eingesetzt werden können, Stellungsisomere (2c, 2d, 3c, 3d) auftreten. Als Folge können dann auch die Verbindungen der Formel 1 als Gemische von zwei Stellungsisomeren (1c und 1d) vorliegen:

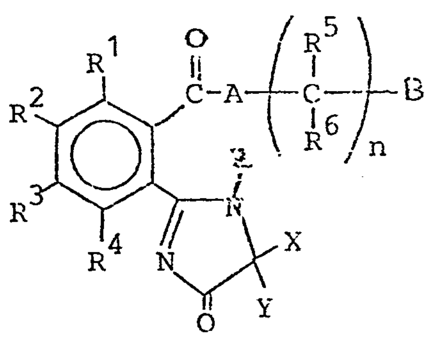

1c

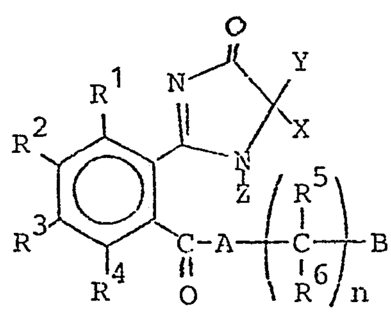

1d

Zur Vedeutlichung der Entstehung der Isomeren mögen zwei Synthesebeispiele für Verbindungen der Formeln 2 und 3 dienen:

 0144749

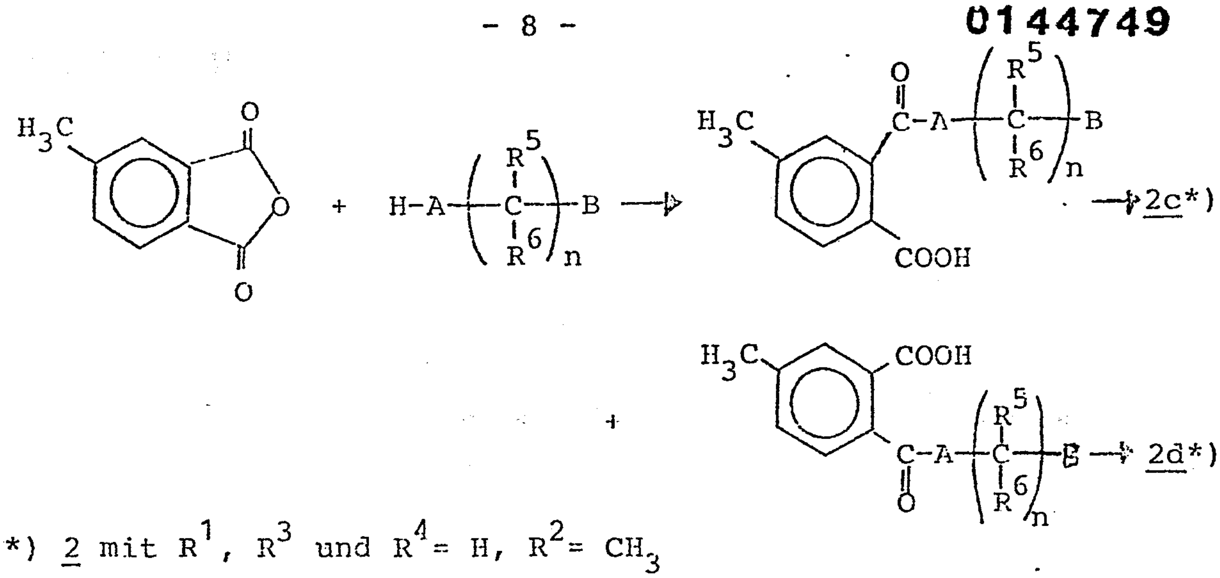

2c*)

2d*)

*) $\underline{2}$ mit $R^1$, $R^3$ und $R^4$ = H, $R^2$ = $CH_3$

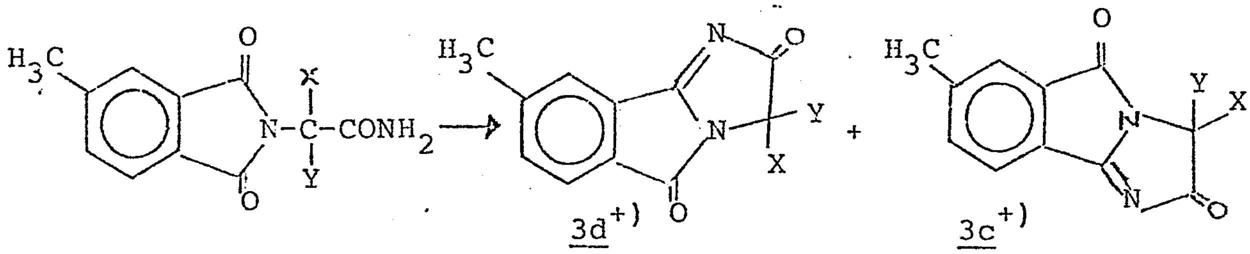

$\underline{3d}$+)

$\underline{3c}$+)

+) $\underline{3}$ mit $R^1$, $R^3$ und $R^4$ = H, $R^2$ = $CH_3$

Die vorliegenden erfindungsgemäßen Verbindungen weisen eine ausgezeichnete herbizide Wirksamkeit gegen ein breites Spektrum wirtschaftlich wichtiger mono- und dikotyler Schadpflanzen auf. Auch schwer bekämpfbare perennierende Wurzelunkräuter werden durch die Wirkstoffe gut erfaßt. Dabei ist es gleichgültig, ob die Substanzen in Vorsaat-, Vorauflauf- oder Nachauflauspritzung ausgebracht werden. Werden die erfindungsgemäßen Verbindungen vor dem Keimen auf die Erdoberfläche appliziert, so wird das Auflaufen der Keimlinge nicht vollständig verhindert. Die Unkräuter wachsen bis zum Keimblattstadium heran, stellen jedoch dann ihr Wachstum ein und sterben schließlich nach 3 Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz

sehr früh und nachhaltig durch den Einsatz der neuen erfindungsgemäßen Mittel beseitigt werden kann. Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z. B. Weizen, Gerste, Roggen, Reis, Mais, Zuckerrübe, Baumwolle und Soja nur unwesentlich oder gar nicht geschädigt. Die erfindungsgemäßen Substanzen besitzen somit eine ausgezeichnete Selektivität bei Kulturpflanzen und eignen sich aus diesen Gründen sehr gut zur Bekämpfung von unerwünschtem Pflanzenwuchs in landwirtschaftlichen Nutzpflanzungen.

Darüber hinaus weisen sie wachstumsregulatorische Eigenschaften bei Kulturpflanzen auf. Sie greifen regulierend in den pflanzeneigenen Stoffwechsel ein und können damit zur Ernteerleichterung wie z. B. durch Auslösen von Desikkation, Abszission und Wuchsstauchung eingesetzt werden. Desweiteren eignen sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Gegenstand der vorliegenden Erfindung sind daher auch herbizide und pflanzenwachstumsregulierende Mittel, welche dem Wirkstoff der Formel 1 neben üblichen Formulierungshilfmitteln enthalten.

Die erfindungsgemäßen Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, z: B. polyoxethylierte Alkylphenole, polyoxethylierte Fettalkohole, Alkyl- oder Alkylphenylsulfonate und Dispergiermittel, z. B. ligninsulfonsaures Natrium, 2,2'-dinaphthylmethan-6,6'-disulfonsaures Natrium, dibutylnaphthalinsulfonsaures Natrium oder auch oleoylmethyltaurinsaures Natrium enthalten. Die Herstellung erfolgt in üblicher Weise, z. B. durch Mahlen und Vermischen der Komponenten.

Emulgierbare Konzentrate können z. B. durch Auflösen des Wirkstoffes in einem inerten organischen Lösungsmittel, z. B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden: Alkyl-arylsulfonsaure Calciumsalze wie Ca-dodecylbenzolsulfonat oder nichtionische Emulgatoren wie Fettsäurepolyglykolester, Alkyl-arylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des Wirkstoffes mit feinverteilten, festen Stoffen, z. B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial

hergestellt werden oder durch Aufbringen von Wirkstoffkonzentrationen mittels Bindemitteln, z. B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise, gewünschtenfalls in Mischung mit Düngemitteln, granuliert werden.

In Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 10 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z. B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Die Aufwandmengen an Wirkstoff der Formel 1 variieren je nach Indikation zwischen 0,01 bis 10 kg Wirkstoff.

Auch Mischungen oder Mischformulierungen mit anderen Wirkstoffen, wie z. B. Insektiziden, Akariziden, Herbiziden, Düngemitteln, Wachstumsregulatoren oder Fungiziden sind gegebenenfalls möglich.

Nachstehend seien einige Formulierungsbeispiele aufgeführt:

Ein Stäubemittel wird erhalten, indem man 10 Gewichtsteile Wirkstoff und 90 Gewichtsteile Talkum oder Inertstoff mischt und in einer Schlagmühle zerkleinert.

Ein in Wasser leicht dispergierbares, benetzbares Pulver wird erhalten, indem man 25 Gewichtsteile Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

Ein in Wasser leicht dispergierbares Dispersionskonzentrat wird erhalten, indem man 20 Gewichtsteile Wirkstoff mit 6 Gewichtsteilen Alkylphenolpolyglykolether (z.B. ®Triton X 207 von Rohm und Haas Co.), 3 Gewichtsteilen Isotridecanolpolyglykolether (8 AeO) und 71 Gewichtsteilen paraffinischem Mineralöl (Siedebereich z. B. ca. 255 bis über 377 °C) mischt und in einer Reibkugelmühle auf eine Feinheit von unter 5 Mikron vermahlt.

Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertes Nonylphenol (10 AeO) als Emulgator.

Die folgenden Beispiele dienen der weiteren Erläuterung der Erfindung:

A. Herstellungsbeispiele

Beispiel 1 (Ausgangsprodukt)

2-(1-Methoxycarbonyl)-ethoxycarbonyl-benzoesäure-N-2-(2-carbamoyl-3-methyl)-butylamid

Zu einer Mischung aus 23,3 g (0,18 mol) 2-Methylvalinamid, 21,7 g Triethylamin und 150 ml Methylenchlorid tropft man bei 0 bis 10 °C 48,4 g (0,18 mol) 2-(1-Methoxycarbonyl)-ethoxycarbonyl-benzoylchlorid zu. Man rührt 1 h bei Raumtemperatur, wäscht das Reaktionsgemisch 2mal mit je 100 ml Wasser, trocknet die organische Phase über Natriumsulfat und dampft ein: 50,1 g (81 % der Theorie) 2-(1-Methoxycarbonyl)-ethoxycarbonyl-benzoesäure-N-2-(2-carbamoyl-3-methyl)-butylamid, ein farbloses Öl.

$^1$H-NMR (60 MHz, $CDCl_3$) $\delta$ = 1,00 (d, J = 7 Hz, 6H, $-CH-(C\underline{H}_3)_2$); 1,60 (s, 3H, $CH_3$); 1,64 (d, J= 7 Hz, 3H, $CH-C\underline{H}_3$); 2,2 (h, J = 7 Hz, 1H, $-C\underline{H}-(CH_3)_2$); 3,83 (s, 3H, $OCH_3$); 5,43 (q, J = 7 Hz, 1H, $C\underline{H}-CH_3$); 6,8 - 8,3 ppm (m, 7H, Ph, NH, $NH_2$)

Beispiel 2

5-Isopropyl-2-(2-<1-methoxycarbonyl>-ethoxycarbonyl-phenyl)-5-methyl-4-oxo-2-imidazolin

Zu einer Lösung von 42,8 g (0,12 mol) 2-(1-Methoxycarbonyl)-ethoxycarbonyl-benzoesäure-N-2-(2-carbamoyl-3-methyl)-butylamid in 50 ml Phosphoroxychlorid gibt

man bei Raumtemperatur portionsweise 27,0 g (0,13 mol) Phosphorpentachlorid zu, rührt 2 h bei Raumtemperatur nach, dampft im Vakuum ein und hydrolysiert mit 300 g Eis. Nach der Neutralisation mit Natriumhydrogencarbonat wird 2mal mit Essigester extrahiert, über Natriumsulfat getrocknet und eingedampft. Man erhält 34,3 g (84 % d. Theorie) 5-Isopropyl-2-(2-<1-methoxycarbonyl>-ethoxycarbonyl-phenyl)-5-methyl-4-oxo-2-imidazolin, ein leicht hellgelbes Öl.

$^1$H-NMR ($CDCl_3$, 60 MHz) $\delta$ = 0,90, 1,05 (2d, J = 7 Hz, 6H, CH-($C\underline{H}_3$)$_2$); 1,37 (s, 3H, $CH_3$); 1,57 (d, J = 7 Hz, 3H, CH-$C\underline{H}_3$); 2,1 (h, J = 7 Hz, 1H, C$\underline{H}$-($CH_3$)$_2$); 3,73 (s, 3H, $OCH_3$); 5,23 (q, J = 7 Hz, 1H, -C$\underline{H}$-$CH_3$); 7,1 - 8,1 ppm (m, 5H, Ph, NH).

Beispiel 3

1-Acetyl-5-isopropyl-2-(2-<1-methoxycarbonyl>-ethoxycarbonylphenyl)-5-methyl-4-oxo-2-imidazolin

Eine Mischung aus 8,9 g (0,026 mol) 5-Isopropyl-2-(2-<1-methoxycarbonyl>-ethoxycarbonylphenyl)-5-methyl-4-oxo-2-imidazolin, 15 g Acetanhydrid und 50 ml abs. Toluol werden 2 h auf 80 °C erhitzt. Nach dem Abkühlen

wird auf 200 g Eis gegossen, die organische Phase abgetrennt, getrocknet und chromatographisch gereinigt (Kieselgel, LM Petrolether - Essigester 7 : 3). Man erhält 6,9 g (69 % der Theorie) 1-Acetyl-5-isopropyl-2-(2-<1-methoxycarbonyl>-ethoxycarbonylphenyl)-5-methyl-4-oxo-2-imidazolin, ein farbloses Öl.

$^1$H-NMR (60 MHz, $CDCl_3$) $\delta$ = 0,97, 1,10 (2d, J = 7 Hz, 6H, $CH-(CH_3)_2$); 1,43 (s, 3H, $CH_3$); 1,56 (d, J = 7 Hz, 3H, $-CH-CH_3$); 2,1 (h, J = 7 Hz, 1H, $CH-(CH_3)_2$); 2,45 (s, 3H, $-COCH_3$); 3,67 (s, 3H, $OCH_3$); 5,2 (q, J = 7 HZ, 1H, $CH-CH_3$); 7,3 - 8,3 ppm (4H, Ph).

Weitere Beispiele sind in Tabelle 1 aufgeführt.

...

Tabelle 1

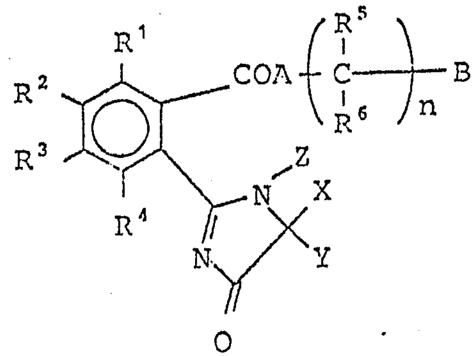

| Bei-spiel Nr. | $R^1$ | $R^2$ | $R^3$ | $R^4$ | $R^5$ | $R^6$ | n | A | B | Z | X | Y | Fp [°C] |
|---|---|---|---|---|---|---|---|---|---|---|---|---|---|
| 4 | H | H | H | H | H | H | 1 | O | $COOCH_3$ | H | $CH_2$ | $CH(CH_3)_2$ | 99-103 |
| 5 | " | " | " | " | " | " | " | " | COOH | " | " | $C_2H_5$ | Harz |
| 6 | " | " | " | " | " | " | " | S | $COOCH_3$ | " | " | $CH(CH_3)_2$ | Oel |
| 7 | " | " | " | " | $CH_3$ | " | " | O | CN | " | " | " | " |
| 8 | " | " | " | " | " | $CH(CH_3)_2$ | " | NH | $CONH_2$ | " | " | " | Harz |
| 9 | " | " | " | " | " | H | " | O | $COOCH_3$ | -CO-Phenyl | " | " | Oel |
| 10 | " | " | Cl | " | " | " | " | " | " | H | " | " | " |
| 11 | " | " | $CH_3$ | " | " | " | " | " | " | " | " | " | " |
| 12 | " | " | H | " | " | " | " | " | $COOC_2H_5$ | " | " | $C_2H_5$ | " |
| 13 | " | " | " | " | " | " | " | " | " | " | " | $CH(CH_3)_2$ | " |
| 14 | " | " | " | " | " | " | " | " | " | " | " | " | Harz (Hydro-chlorid) |

...

Fortsetzung Tabelle 1

| Bei-spiel Nr. | $R_1$ | $R_2$ | $R_3$ | $R_4$ | $R_5$ | $R_6$ | n | A | B | Z | X | Y | Fp [°C] |
|---|---|---|---|---|---|---|---|---|---|---|---|---|---|
| 15 | H | H | H | H | $CH_3$ | H | 1 | O | $COOC_2H_5$ | $-COOCH_3$ | $CH_3$ | $CH(CH_3)_2$ | Oel |
| 16 | " | " | " | " | " | " | " | " | " | $-CONHCH_3$ | " | " | " |
| 17 | " | " | " | " | " | " | " | " | " | 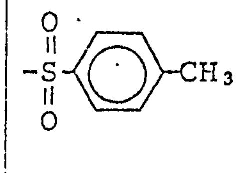 | " | " | " |
| 18 | " | " | " | " | " | " | " | " | " | H | $-CH_2-CH_2-CH_2-CH_2-CH(CH_3)-$ | | Oel |
| 19 | " | " | " | " | " | " | " | " | " | $-COCH(CH_3)_2$ | | " | Oel |
| 20 | " | " | " | " | " | $CH_3$ | " | " | CN | H | $CH_3$ | $CH_3$ | " |
| 21 | " | " | " | " | " | H | " | " | $COOC_2H_5$ | " | " | $CH_2-CH(CH_3)_2$ | " |
| 22 | " | " | " | " | " | " | " | " | " | " | " | $CH(CH_3)-C_2H_5$ | " |
| 23 | " | " | " | " | " | " | " | " | " | $-COOCH_3$ | " | " | " |
| 24 | " | " | " | " | " | " | " | " | " | $-CONHCH_3$ | " | " | " |
| 25 | $-CH=CH-CH=CH-$ | | | " | " | " | " | " | $COOCH_3$ | H | " | $CH(CH_3)_2$ | 51-3 |
| 26 | " | " | | " | " | " | " | " | " | $-CONHCH_3$ | " | " | Harz |

Fortsetzung Tabelle 1

| Beispiel Nr. | $R_1$ | $R_2$ | $R_3$ | $R_4$ | $R_5$ | $R_6$ | n | A | B | Z | X | Y | Fp [°C] |
|---|---|---|---|---|---|---|---|---|---|---|---|---|---|
| 27 | H | $CH_3$ | H | H | H | H | 1 | O | $COOCH_3$ | H | $CH_3$ | $CH(CH_3)_2$ | Öl |
| 28 | " | " | " | " | " | " | " | " | " | $COCH_3$ | " | " | " |
| 29 | " | " | " | " | " | " | " | " | " | $COOCH_3$ | " | " | " |
| 30 | " | " | " | " | " | " | " | " | " | $COCOOCH_3$ | " | " | " |
| 31 | " | " | " | " | " | " | " | " | " | $CONHCH_3$ | " | " | " |
| 32 | " | " | " | " | " | " | " | " | " | CONH-Phenyl | " | " | " |
| 33 | " | " | " | " | " | " | " | " | " | $CH_3$ | " | " | " |
| 34 | " | " | " | " | " | " | " | " | " | $COCH_2$-Phenyl | " | " | " |
| 35 | " | " | " | " | " | " | " | " | $COOC_2H_5$ | $COCH_3$ | " | " | " |
| 36 | " | " | " | " | " | " | " | " | " | $COOCH_3$ | " | " | " |
| 37 | " | " | " | " | " | " | " | " | " | $CONHCH_3$ | " | " | " |
| 38 | " | " | " | " | " | " | " | " | " | $CH_2-COOCH_3$ | " | " | " |
| 39 | " | " | " | " | " | " | " | " | " | $CONHNH_2$ | " | " | " |
| 40 | " | " | " | " | " | " | " | " | " | $CONHN(CH_3)_2$ | " | " | " |

Fortsetzung Tabelle 1

| Beispiel Nr. | $R_1$ | $R_2$ | $R_3$ | $R_4$ | $R_5$ | $R_6$ | n | A | B | Z | X | Y | Fp [°C] |
|---|---|---|---|---|---|---|---|---|---|---|---|---|---|
| 41 | H | $CH_3$ | H | H | H | $CH_3$ | 1 | O | $COOC_2H_5$ | H | $CH_3$ | $CH(CH_3)_2$ | Öl |
| 42 | " | " | " | " | " | " | " | " | " | $COCOOC_3H_7$ | " | " | " |
| 43 | " | H | " | $CH_3$ | $CH_3$ | H | " | " | " | H | " | " | " |
| 44 | " | " | " | " | " | " | " | " | " | $CONHCH_3$ | " | " | " |
| 45 | " | " | " | " | " | " | " | " | " | $COOC_4H_9$ | " | " | " |
| 46 | " | Cl | Cl | H | " | " | " | " | " | H | " | " | " |
| 47 | " | " | " | " | " | " | " | " | " | $COCH_3$ | " | " | " |
| 48 | " | " | " | " | " | " | " | " | " | $COCOOCH_3$ | " | " | " |
| 49 | " | " | $CH_3$ | " | " | " | " | " | $COOCH_3$ | H | " | cyclopropyl | " |
| 50 | " | " | " | " | " | " | " | " | " | CONH-Phenyl | " | " | " |

Die Verbindungen 27 bis 40, 43 bis 45, 49 und 50 liegen jeweils als Gemisch der Stellungsisomeren gemäß S. 7 (Formeln 1c und 1d) vor.

0144749

## B. Biologische Beispiele

### Prüfung auf herbizide Wirkung

Die Schädigung der Unkrautpflanzen bzw. die Kulturpflanzenverträglichkeit wurde in einem Schlüssel von 0 - 5 bonitiert.
Dabei bedeutet

0 = ohne Wirkung (Schaden)
1 = 0 - 20 % Wirkung
2 = 20 - 40 % Wirkung
3 = 40 - 60 % Wirkung
4 = 60 - 80 % Wirkung
5 = 80 - 100 % Wirkung

### 1. Wirkung gegen Unkräuter

Samen bzw. Rhizomstücke mono- und dikotyler Unkräuter wurden in Lehmerde in Plastiktöpfen (Ø 9 cm) ausgelegt und mit Erde abgedeckt. Die als benetzbare Pulver bzw. als Emulsionskonzentrate formulierten erfindungsgemäßen Verbindungen wurden in Form wäßriger Suspensionen bzw. Emulsionen auf die Erdoberfläche appliziert. Die Wasseraufwandmenge pro Topf entsprach dabei umgerechnet 600 l/ha. Nach der Behandlung wurden die Versuchstöpfe im Gewächshaus aufgestellt und die Versuchspflanzen unter guten Wachstumsbedingungen (Temperatur: 23 ± 1 °C; rel. Luftfeuchte 60 - 80 %) kultiviert. Nach ca. 3 Wochen wurde die Pflanzenschädigung visuell bonitiert. Als Vergleich dienten dabei unbehandelte Kontrollen.
Wie aus den Werten der Tabelle 2 hervorgeht, weisen

die erfindungsgemäßen Verbindungen im Vorauflaufverfahren eine zum Teil ausgezeichnete herbizide Wirksamkeit gegen wirtschaftlich bedeutende mono- und dikotyle Schadpflanzen auf.

In ähnlicher Weise werden verschiedene Unkräuter in Töpfen im Gewächshaus bis zum 3-6-Blattstadium herangezogen und dann im Nachauflaufverfahren mit den erfindungsgemäßen Verbindungen (formuliert als Spritzpulver) behandelt. 4 Wochen später wurden die Versuchspflanzen im Vergleich zu unbehandelten Kontrollpflanzen visuell bonitiert, indem die Schädigung geschätzt wurde.
Die Ergebnisse des Versuchs (Tabelle 3) belegen die guten herbiziden Eigenschaften der Verbindungen.

Tabelle 2

Herbizide Wirksamkeit im Vorauflaufverfahren (% Schädigung)

| Beispiel Nr. | kg AS /ha | ALM | POA | AMR | SIA | TAW | GS |
|---|---|---|---|---|---|---|---|
| 2 | 2,4 | 5 | 5 | 5 | 5 | 5 | 5 |
| 3 | 2,4 | 5 | 5 | 5 | 5 | 5 | 5 |
| 4 | 2,4 | 5 | 5 | 5 | 5 | 5 | 5 |
| 9 | 2,4 | 5 | 4 | 5 | 5 | 5 | 5 |
| 10 | 2,4 | 5 | 5 | 5 | 5 | - | - |
| 12 | 2,4 | 5 | 5 | 5 | 5 | 4 | 5 |
| 13 | 2,4 | 5 | 5 | 5 | 5 | 5 | 5 |
| 14 | 2,4 | 5 | 5 | 5 | 5 | 5 | 5 |
| 15 | 2,4 | 5 | 5 | 5 | 5 | 4 | 4 |
| 16 | 2,4 | 5 | 5 | 5 | 5 | 5 | 5 |
| 25 | 2,4 | 5 | 5 | 5 | 5 | - | - |
| 26 | 2,4 | 4 | - | 5 | 5 | - | - |
| 27 | 2,4 | 5 | 5 | 5 | 5 | 5 | 4 |
| 31 | 2,4 | 5 | 5 | 5 | 5 | - | - |

Tabelle 2 (Fortsetzung)

Herbizide Wirksamkeit im Vorlaufverfahren (% Schädigung)

| Beispiel Nr. | kg AS /ha | LOM | ECG | STM | SIA |
|---|---|---|---|---|---|
| 28 | 2,4 | 5 | 5 | 5 | 5 |
| 29 | 2,4 | 5 | 5 | 5 | 5 |
| 35 | 2,4 | 5 | 5 | 5 | 5 |
| 36 | 2,4 | 4 | - | 4 | 5 |
| 37 | 2,4 | 4 | 5 | 5 | 5 |

ALM = Alopecurus myosuroides
POA = Poa annua
AMR = Amaranthus retroflexus
SIA = Sinapis alba
TAW = Triticum aestivum (Winter-)
GS = Glycine soja
LOM = Lolium multiflorum
ECG = Echinochloa crus-galli
STM = Stellaria media

Tabelle 3

Herbizide Wirksamkeit im Nachlaufverfahren (% Schädigung)

| Beispiel-Nr. | kg AS/ha | ALM | POA | AMR | SIA | LOM | ECG | STM |
|---|---|---|---|---|---|---|---|---|
| 2 | 2,4 | - | - | 5 | 5 | - | - | - |
| 4 | 2,4 | 5 | 4 | 5 | 5 | - | - | - |
| 10 | 2,4 | 4 | - | 5 | 5 | - | - | - |
| 12 | 2,4 | - | - | 5 | 4 | - | - | - |
| 14 | 2,4 | - | - | 5 | 5 | - | - | - |
| 16 | 2,4 | - | - | 5 | 5 | - | - | - |
| 27 | 2,4 | 5 | 4 | 5 | 5 | - | - | - |
| 28 | 2,4 | 5 | 5 | 5 | 5 | - | - | - |
| 29 | 2,4 | - | - | - | 5 | 5 | 5 | 5 |
| 31 | 2,4 | - | - | 5 | 5 | - | - | - |
| 35 | 2,4 | - | - | - | 5 | 5 | 5 | 5 |
| 36 | 2,4 | - | - | - | 5 | 4 | - | 4 |
| 37 | 2,4 | - | - | - | 4 | - | - | - |

0144749

## Prüfung auf wachstumsregulierende Wirkung

### 1. Wuchshemmung bei Getreide

In Schalenversuchen im Gewächshaus wurden junge Getreidepflanzen (Weizen, Gerste und Roggen) im 3-Blattstadium mit den zu prüfenden Verbindungen tropfnaß gespritzt. Nachdem die unbehandelten Kontrollpflanzen eine Wuchshöhe von etwa 55 cm erreicht hatten, wurde bei allen Pflanzen der Zuwachs gemessen und die Wuchshemmung in % des Zuwachses der Kontrollpflanzen berechnet. Es wurde außerdem die phytotoxische Wirkung der Verbindungen beobachtet. Die Verbindungen erwiesen sich als gut wirksam.

### 2. Wuchshemmung an Buschbohnen

10 - 15 cm Buschbohnen wurden mit den Wirkstoffzubereitungen tropfnaß bespritzt. Nach 2 Wochen wurde der Zuwachs gemessen und die Wuchshemmung in % des Zuwachses der Kontrollpflanzen berechnet. Die Verbindungen erwiesen sich als gut wirksam.

die erfindungsgemäßen Verbindungen im Vorauflaufverfahren eine zum Teil ausgezeichnete herbizide Wirksamkeit gegen wirtschaftlich bedeutende mono- und dikotyle Schadpflanzen auf.

In ähnlicher Weise wurden verschiedene Unkräuter in Töpfen im Gewächshaus bis zum 3-6-Blattstadium herangezogen und dann im Nachauflaufverfahren mit den erfindungsgemäßen Verbindungen (formuliert als Spritzpulver) behandelt. 4 Wochen später wurden die Versuchspflanzen im Vergleich zu unbehandelten Kontrollpflanzen visuell bonitiert, indem die Schädigung geschätzt wurde.
Die Ergebnisse des Versuchs (Tabelle 3) belegen die guten herbiziden Eigenschaften der Verbindungen.

Tabelle 2

Herbizide Wirksamkeit im Vorauflaufverfahren (% Schädigung)

| Beispiel Nr. | kg AS /ha | ALM | POA | AMR | SIA | TAW | GS |
|---|---|---|---|---|---|---|---|
| 2 | 2,4 | 5 | 5 | 5 | 5 | 5 | 5 |
| 3 | 2,4 | 5 | 5 | 5 | 5 | 5 | 5 |
| 4 | 2,4 | 5 | 5 | 5 | 5 | 5 | 5 |
| 9 | 2,4 | 5 | 4 | 5 | 5 | 5 | 5 |
| 10 | 2,4 | 5 | 5 | 5 | 5 | - | - |
| 12 | 2,4 | 5 | 5 | 5 | 5 | 4 | 5 |
| 13 | 2,4 | 5 | 5 | 5 | 5 | 5 | 5 |
| 14 | 2,4 | 5 | 5 | 5 | 5 | 5 | 5 |
| 15 | 2,4 | 5 | 5 | 5 | 5 | 4 | 4 |
| 16 | 2,4 | 5 | 5 | 5 | 5 | 5 | 5 |
| 25 | 2,4 | 5 | 5 | 5 | 5 | - | - |
| 26 | 2,4 | 4 | - | 5 | 5 | - | - |
| 27 | 2,4 | 5 | 5 | 5 | 5 | 5 | 4 |
| 31 | 2,4 | 5 | 5 | 5 | 5 | - | - |

Tabelle 2 (Fortsetzung)

Herbizide Wirksamkeit im Vorauflaufverfahren (% Schädigung)

| Beispiel Nr. | kg AS /ha | LOM | ECG | STM | SIA |
|---|---|---|---|---|---|
| 28 | 2,4 | 5 | 5 | 5 | 5 |
| 29 | 2,4 | 5 | 5 | 5 | 5 |
| 35 | 2,4 | 5 | 5 | 5 | 5 |
| 36 | 2,4 | 4 | – | 4 | 5 |
| 37 | 2,4 | 4 | 5 | 5 | 5 |

ALM = Alopecurus myosuroides
POA = Poa annua
AMR = Amaranthus retroflexus
SIA = Sinapis alba
TAW = Triticum aestivum (Winter-)
GS = Glycine soja
LOM = Lolium multiflorum
ECG = Echinochloa crus-galli
STM = Stellaria media

Tabelle 3

Herbizide Wirksamkeit im Nachauflaufverfahren (% Schädigung)

| Beispiel-Nr. | kg AS/ha | ALM | POA | AMR | SIA | LOM | ECG | STM |
|---|---|---|---|---|---|---|---|---|
| 2 | 2,4 | – | – | 5 | 5 | – | – | – |
| 4 | 2,4 | 5 | 4 | 5 | 5 | – | – | – |
| 10 | 2,4 | 4 | – | 5 | 5 | – | – | – |
| 12 | 2,4 | – | – | 5 | 4 | – | – | – |
| 14 | 2,4 | – | – | 5 | 5 | – | – | – |
| 16 | 2,4 | – | – | 5 | 5 | – | – | – |
| 27 | 2,4 | 5 | 4 | 5 | 5 | – | – | – |
| 28 | 2,4 | 5 | 5 | 5 | 5 | – | – | – |
| 29 | 2,4 | – | – | – | 5 | 5 | 5 | 5 |
| 31 | 2,4 | – | – | 5 | 5 | – | – | – |
| 35 | 2,4 | – | – | – | 5 | 5 | 5 | 5 |
| 36 | 2,4 | – | – | – | 5 | 4 | – | 4 |
| 37 | 2,4 | – | – | – | 4 | – | – | – |

Prüfung auf wachstumsregulierende Wirkung

1. Wuchshemmung bei Getreide

In Schalenversuchen im Gewächshaus wurden junge Getreidepflanzen (Weizen, Gerste und Roggen) im 3-Blattstadium mit den zu prüfenden Verbindungen tropfnaß gespritzt. Nachdem die unbehandelten Kontrollpflanzen eine Wuchshöhe von etwa 55 cm erreicht hatten, wurde bei allen Pflanzen der Zuwachs gemessen und die Wuchshemmung in % des Zuwachses der Kontrollpflanzen berechnet. Es wurde außerdem die phytotoxische Wirkung der Verbindungen beobachtet. Die Verbindungen erwiesen sich als gut wirksam.

2. Wuchshemmung an Buschbohnen

10 - 15 cm Buschbohnen wurden mit den Wirkstoffzubereitungen tropfnaß bespritzt. Nach 2 Wochen wurde der Zuwachs gemessen und die Wuchshemmung in % des Zuwachses der Kontrollpflanzen berechnet. Die Verbindungen erwiesen sich als gut wirksam.

Patentansprüche:

1. Imidazolinone der Formel 1

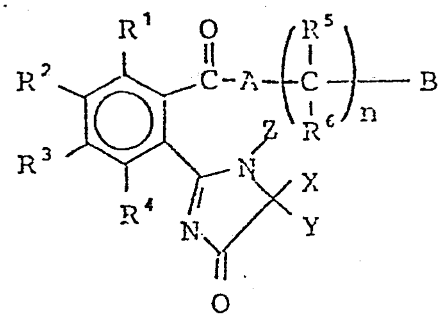

1

in welcher

A O, S, NH oder $N(C_1-C_6)$Alkyl;

B $-CN$, $-COOR^7$, $-CONR^8R^9$ oder $-COSR^{10}$;

X $(C_1-C_4)$Alkyl;

Y $(C_1-C_6)$Alkyl, Cyclo$(C_3-C_6)$Cycloalkyl, $(C_2-C_4)$Alkenyl, $(C_2-C_4)$Alkinyl, Phenyl oder Benzyl;

X und Y zusammen mit dem Kohlenstoffatom, an dem sie gebunden sind, eine gegebenenfalls durch $-CH_3$ substituierte Spiro-cyclo$(C_3-C_6)$alkylgruppe;

Z Wasserstoff, $(C_1-C_4)$Alkyl, welches durch $(C_1-C_4)$Alkoxycarbonyl substituiert sein kann, $(C_3-C_4)$Alkenyl, Propargyl, $-CO-R^{11}$ oder $-SO_2-R^{12}$;

n die Zahlen 1, 2 oder 3;

$R^1$, $R^2$, $R^3$ und $R^4$ unabhängig voneinander Wasserstoff, $(C_1-C_4)$Alkyl, $(C_1-C_6)$Alkoxy, $(C_1-C_6)$Alkoxycarbonyl, Halogen, Nitro, Cyano, Phenoxy oder Phenyl, das gegebenenfalls mit $(C_1-C_4)$Alkyl, $(C_1-C_4)$Alkoxy oder Halogen substituiert ist, und jeweils zwei benachbarte Reste $R^1$, $R^2$, $R^3$ oder $R^4$ gemeinsam die Gruppierung $-CH=CH-CH=CH-$ bilden können;

$R^5$ und $R^6$ unabhängig voneinander Wasserstoff, $(C_1-C_4)$-Alkyl oder Phenyl;

$R^7$ Wasserstoff, $(C_1-C_{12})$Alkyl, welches gegebenenfalls bis zu zweimal, vorzugsweise jedoch einfach mit $(C_1-C_4)$Alkoxy, $(C_1-C_4)$Alkoxyethoxy, Cyclo$(C_3-C_6)$alkyl, Benzyloxy, Phenyl, Halogen, Cyano, Hydroxyl, Oxiranyl, Tetrahydrofuryl, $(C_1-C_4)$Alkylamino, Di$(C_1-C_4)$alkylamino, $(C_1-C_4)$Alkylthio, $(C_1-C_4)$Alkylsulfinyl, $(C_1-C_4)$Alkylsulfonyl, Triazolyl oder Imidazolyl substituiert ist; $(C_3-C_6)$Alkenyl, Halogen$(C_3-C_6)$-alkenyl, Cyclo$(C_3-C_6)$alkyl, Cyclo$(C_5-C_6)$alkenyl, $(C_3-C_6)$Alkinyl, Benzyl, welches gegebenenfalls im Benzolkern durch Chlor oder Methyl substituiert sein kann, Phenyl, welches gegebenenfalls mit bis zu zwei $(C_1-C_4)$Alkyl-, Nitro-, Halogen- oder Methoxygruppen substituiert sein kann, oder die Gruppe

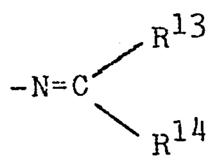

$R^8$ Wasserstoff oder $(C_1-C_4)$Alkyl;

$R^9$ Wasserstoff, $(C_1-C_6)$Alkyl, welches gegebenenfalls mit einer Hydroxygruppe oder einer $(C_1-C_3)$Alkoxygruppe substituiert ist, $(C_1-C_4)$Alkylsulfonyl oder Halogen$(C_1-C_4)$alkylsulfonyl;

$R^8$ und $R^9$ gemeinsam mit dem Stickstoffatom einen 5- oder 6-gliedrigen Ring, in dem ein Kohlenstoffatom durch Sauerstoff oder Stickstoff ersetzt sein kann und der gegebenenfalls mit bis zu zwei Methylgruppen substituiert ist;

$R^{10}$ $(C_1-C_6)$Alkyl, Phenyl oder $(C_1-C_4)$Alkoxycarbonyl-$(C_1-C_2)$alkyl;

$R^{11}$ $(C_1-C_{12})$Alkyl, das gegebenenfalls mit bis zu zwei $(C_1-C_4)$Alkoxygruppen oder mit bis zu drei Halogen substituiert ist; Phenyl, das mit bis zu zwei Halogen, einer Methyl-, einer Nitro- oder einer Methoxygruppe substituiert sein kann; $(C_1-C_4)$Alkoxy, Cyclo$(C_3-C_7)$alkyl, $(C_1-C_4)$Alkoxycarbonyl, Benzyloxy, Phenoxy oder $-NR^{15}R^{16}$;

$R^{12}$ $(C_1-C_4)$Alkyl, $CF_3$, $CCl_3$, Phenyl, Chlorphenyl oder Methylphenyl;

$R^{13}$ und $R^{14}$ unabhängig voneinander Wasserstoff, $(C_1-C_4)$-Alkyl, Alkoxy$(C_1-C_4)$alkyl, Cyclo$(C_3-C_6)$alkyl oder Phenyl, oder gemeinsam mit dem Kohlenstoffatom, an dem sie gebunden sind, eine Cyclo$(C_5-C_6)$alkylgruppe;

$R^{15}$ Wasserstoff oder $(C_1-C_4)$Alkyl, und

$R^{16}$ $(C_1-C_4)$Alkyl, Phenyl, Chlorphenyl, Methylphenyl, Amino, Mono- oder Di$(C_1-C_4)$alkylamino

bedeuten, sowie deren optische Isomeren (falls $X \neq Y$ und/oder $R^5 \neq R^6$) sowie ihre Salze mit Säuren und Basen.

2. Verfahren zur Herstellung von Verbindungen der Formel 1 in Anspruch 1, dadurch gekennzeichnet, daß man

a) Amide der Formel 2

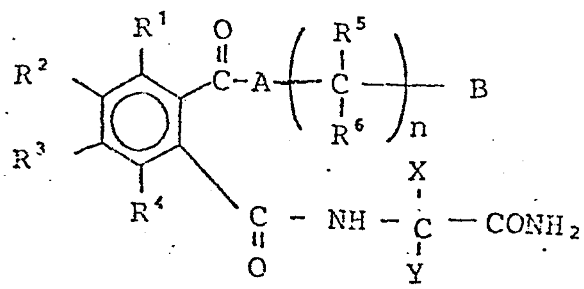

2

durch Wasserabspaltung cyclisiert oder

b) Verbindungen der Formel 3,

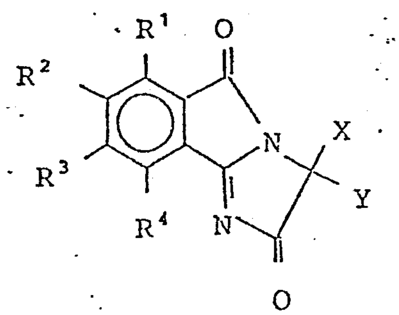

3

mit mindestens äquimolaren Mengen der Verbindungen der Formel 4

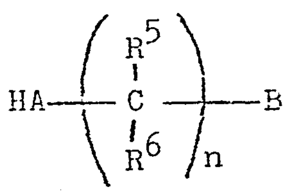

4

umsetzt

und gewünschtenfalls die erhaltenen Verbindungen der Formel 1 mit Z= Wasserstoff durch Alkylierung, Acylierung, Sulfonierung, Oxidation, Verseifung, Veresterung oder Salzbildung in andere Verbindungen der Formel 1 überführt.

3. Verwendung der Verbindungen der Formel 1 als Herbizide und Wachstumsregulatoren.

4. Herbizide und wachstumsregulierende Mittel, dadurch gekennzeichnet, daß sie eine Verbindung der allgemeinen Formel 1 enthalten.

5. Verfahren zur Bekämpfung von unerwünschten Pflanzen bzw. Wachstumsregulation, dadurch gekennzeichnet, daß man eine wirksame Menge einer Verbindung der Formel 1 auf die zu behandelnde Aufbaufläche bzw. die zu behandelnden Pflanzen aufbringt.

2-arylsubstituierte Imidazolinone, Verfahren zu ihrer Herstellung und ihre Verwendung im Pflanzenschutz

Die vorliegende Erfindung betrifft neue, herbizid wirksame 2-arylsubstituierte Imidazolinone der Formel 1

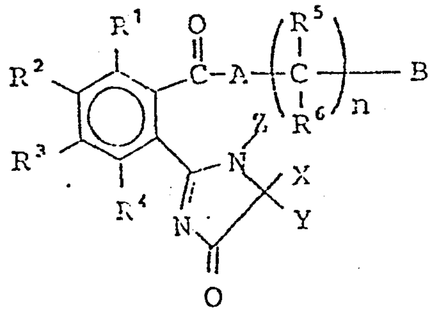

1

in welcher

A O, S, NH oder N($C_1$-$C_6$)-Alkyl);

B -CN, $-COOR^7$, $-CONR^8R^9$ oder $-COSR^{10}$;

X ($C_1$-$C_4$)Alkyl;

Y ($C_1$-$C_6$)Alkyl, Cyclo($C_3$-$C_6$)alkyl, ($C_2$-$C_4$)Alkenyl, ($C_2$-$C_4$)Alkinyl, Phenyl oder Benzyl;

X und Y zusammen mit dem Kohlenstoffatom, an dem sie gebunden sind, eine gegebenenfalls durch $-CH_3$ substituierte Spiro-cyclo($C_3$-$C_6$)alkylgruppe;

Z Wasserstoff, ($C_1$-$C_4$)Alkyl, welches durch ($C_1$-$C_4$)Alkoxycarbonyl substituiert sein kann, ($C_3$-$C_4$)Alkenyl, Propargyl, $-CO-R^{11}$ oder $-SO_2-R^{12}$;

n die Zahlen 1, 2 oder 3;

$R^1$, $R^2$, $R^3$ und $R^4$ unabhängig voneinander Wasserstoff, ($C_1$-$C_4$)Alkyl, ($C_1$-$C_6$)Alkoxy, ($C_1$-$C_6$)Alkoxycarbonyl, Halogen, Nitro, Cyano, Phenoxy oder Phenyl, das gegebenenfalls mit ($C_1$-$C_4$)Alkyl, ($C_1$-$C_4$)Alkoxy oder Halogen substituiert ist, und jeweils zwei benachbarte Reste $R^1$, $R^2$, $R^3$ oder $R^4$ gemeinsam die Gruppierung -CH=CH-CH=CH- bilden können;

$R^5$ und $R^6$ unabhängig voneinander Wasserstoff, $(C_1-C_4)$-Alkyl oder Phenyl;

$R^7$ Wasserstoff, $(C_1-C_{12})$Alkyl, welches gegebenenfalls bis zu zweimal, vorzugsweise jedoch einfach mit $(C_1-C_4)$Alkoxy, $(C_1-C_4)$Alkoxyethoxy, Cyclo$(C_3-C_6)$alkyl, Benzyloxy, Phenyl, Halogen, Cyano, Hydroxyl, Oxiranyl, Tetrahydrofuryl, $(C_1-C_4)$-Alkylamino, Di$(C_1-C_4)$alkylamino, $(C_1-C_4)$Alkylthio, $(C_1-C_4)$Alkylsulfinyl, $(C_1-C_4)$Alkylsulfonyl, Triazolyl oder Imidazolyl substituiert ist; $(C_3-C_6)$Alkenyl, Halogen$(C_3-C_6)$-alkenyl, Cyclo$(C_3-C_6)$alkyl, Cyclo$(C_5-C_6)$alkenyl, $(C_3-C_6)$Alkinyl, Benzyl, welches gegebenenfalls im Benzolkern durch Chlor oder Methyl substituiert sein kann, Phenyl, welches gegebenenfalls mit bis zu zwei $(C_1-C_4)$Alkyl-, Nitro-, Halogen- oder Methoxygruppen substituiert sein kann, oder die Gruppe

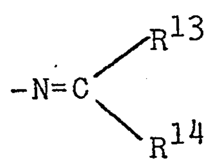

;

$R^8$ Wasserstoff oder $(C_1-C_4)$Alkyl;

$R^9$ Wasserstoff, $(C_1-C_6)$Alkyl, welches gegebenenfalls mit einer Hydroxygruppe oder einer $(C_1-C_3)$Alkoxygruppe substituiert ist; $(C_1-C_4)$Alkylsulfonyl oder Halogen$(C_1-C_4)$alkylsulfonyl;

$R^8$ und $R^9$ gemeinsam mit dem Stickstoffatom einen 5- oder 6-gliedrigen Ring, in dem ein Kohlenstoffatom durch Sauerstoff oder Stickstoff ersetzt sein kann und der gegebenenfalls mit bis zu zwei Methylgruppen substituiert ist;

$R^{10}$ $(C_1-C_6)$Alkyl, Phenyl oder $(C_1-C_4)$Alkoxycarbonyl-$(C_1-C_2)$alkyl;

$R^{11}$ $(C_1-C_{12})$Alkyl, das gegebenenfalls mit bis zu zwei $(C_1-C_4)$Alkoxygruppen oder mit bis zu drei Halogen substituiert ist; Phenyl, das mit bis zu zwei Halogen, einer Methyl-, einer Nitro- oder einer Methoxygruppe substituiert sein kann; $(C_1-C_4)$Alkoxy, Cyclo$(C_3-C_7)$alkyl, $(C_1-C_4)$Alkoxycarbonyl, Benzyloxy, Phenoxy oder $-NR^{15}R^{16}$;

$R^{12}$ $(C_1-C_4)$Alkyl, $CF_3$, $CCl_3$, Phenyl, Chlorphenyl oder Methylphenyl;

$R^{13}$ und $R^{14}$ unabhängig voneinander Wasserstoff, $(C_1-C_4)$-Alkyl, Alkoxy$(C_1-C_4)$alkyl, Cyclo$(C_3-C_6)$alkyl oder Phenyl, oder gemeinsam mit dem Kohlenstoffatom, an dem sie gebunden sind, eine Cyclo$(C_5-C_6)$alkylgruppe;

$R^{15}$ Wasserstoff oder $(C_1-C_4)$Alkyl, und

$R^{16}$ $(C_1-C_4)$Alkyl, Phenyl, Chlorphenyl, Methylphenyl Amino, Mono- oder Di$(C_1-C_4)$alkylamino

bedeuten, sowie deren optische Isomeren (falls X ≠ Y und/oder $R^5$ ≠ $R^6$) sowie ihre Salze mit Säuren und Basen, dadurch gekennzeichnet, daß man

a) Amide der Formel 2

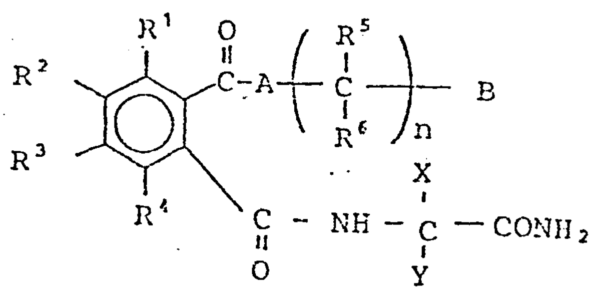

2

durch Wasserabspaltung cyclisiert oder

b) Verbindungen der Formel 3,

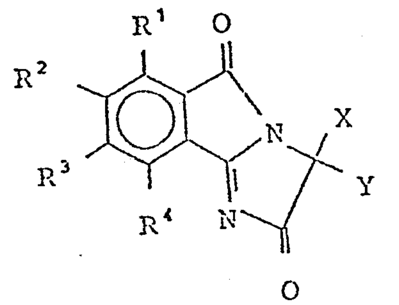

3

mit mindestens äquimolaren Mengen der Verbindungen der Formel 4

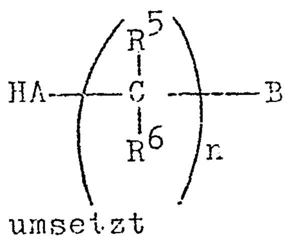

4

umsetzt

und gewünschtenfalls die erhaltenen Verbindungen der Formel 1 mit Z= Wasserstoff durch Alkylierung, Acylierung, Sulfonierung, Oxidation, Verseifung, Veresterung oder Salzbildung in andere Verbindungen der Formel 1 überführt.

2. Verwendung der Verbindungen der Formel 1 als Herbizide und Wachstumsregulatoren.

3. Herbizide und wachstumsregulierende Mittel, dadurch gekennzeichnet, daß sie eine Verbindung der allgemeinen Formel 1 enthalten.

4. Verfahren zur Bekämpfung von unerwünschten Pflanzen bzw. Wachstumsregulatoren, dadurch gekennzeichnet, daß man eine wirksame Menge einer Verbindung der Formel 1 auf die zu behandelnde Aufbaufläche bzw. die zu behandelnden Pflanzen aufbringt.